# EUROPEAN PATENT APPLICATION

(11) **EP 4 564 356 A1**
(43) Date of publication of application: **04.06.2025**
(21) Application number: 22953206.4
(22) Date of filing: 29.07.2022
(51) Int. Cl.: G16B 50/00

(54) **ANTIBODY SELECTION SYSTEM AND OPERATION METHOD THEREFOR**

(71) Applicant: NIKON CORPORATION, Tokyo 140-8601 (JP)
(72) Inventor: TAKATSUKA, Kenji, Tokyo 140-8601 (JP); MOROZUMI, Akihiko, Tokyo 140-8601 (JP)
(74) Representative: Hoffmann Eitle
(86) International application number: PCT/JP2022/029403
(87) International publication number: WO 2024/024118

(57) **Abstract**

A embodiment of the present invention is a working process of a system for selecting an antibody, the system being communicatively connected to an antibody information server, the system having a control unit and a display device, the working process comprising following steps executed by the control unit:
a first step of displaying, on the display device,
a microscopic image of biological tissue or cell;
a nucleotide sequence of a nucleic acid comprised in the biological tissue or cell;
data indicating a characteristics of the nucleic acid or a characteristics of the nucleotide sequence; and
name(s) of one or more genes obtained based on the data,

a second step of transmitting an ID of a gene selected from the one or more genes to the antibody information server,
a third step of receiving information on an antibody against the protein encoded by the selected gene from the antibody information server,
a fourth step of displaying the antibody information on the display device.

## Description

### [technical field]

This invention relates to a system for selecting antibodies and a working process of the system.

### [Background art]

When a disease-causing gene is identified, researchers use antibody staining or immunoprecipitation to confirm the expression of the protein encoded by the gene and examine the function of the gene. In this process, it is necessary to determine whether the expression of the disease-causing e gene and other related genes is increased or decreased when compared to the surrounding normal tissue.

At that time,
1) Which genes are associated with the disease-causing gene?
2) When the relevant genes are found, are antibodies suitable for antibody staining or immunoprecipitation available?
3) Which manufacturer's antibodies are suitable?
4) Which antibody should be selected from those of the same manufacturer?
(5) When a certain antibody is used, whether the staining is successful, etc.

Such information is often not available only from the website of the antibody manufacturer.

### [Summary]

### [Problem to be solved by the invention]

The subject of this invention is to provide a system for easily selecting antibodies and a method for operating the system.

### [Means for solving the problem]

An aspect of the present invention is a working process of a system for selecting an antibody, the system being communicatively connected to an antibody information server, the system having a control unit and a display device, the working process comprising following steps executed by the control unit: a first step of displaying, on the display device, a microscopic image of biological tissue or cell; a nucleotide sequence of a nucleic acid comprised in the biological tissue or cell; data indicating a characteristics of the nucleic acid or a characteristics of the nucleotide sequence; and name(s) of one or more genes obtained based on the data, a second step of transmitting an ID of a gene selected from the one or more genes to the antibody information server, a third step of receiving information on an antibody against the protein encoded by the selected gene from the antibody information server, a fourth step of displaying the antibody information on the display device.

The other aspects are to be made clear from the following description of the embodiments of the present invention.

### [Brief description of drawings]

[Fig. 1] Fig. 1 illustrates a flowchart showing a working process of a system for selecting antibodies in one embodiment of the invention,.
[Fig. 2] Fig. 2 illustrates a schematic diagram the overall picture including a system for selecting antibodies as well as other systems related to said system and an external server.
[Fig. 3] Fig. 3 illustrates an example of the relationship between the system for selecting antibodies and an external server in one embodiment of the present invention.
[Fig. 4] Fig. 4 illustrates an example of the relationship between the system for selecting antibodies and an external server in one embodiment of the present invention.
[Fig. 5] Fig. 5 illustrates an example of the relationship between the system for selecting antibodies and an external server in one embodiment of the present invention.
[Fig. 6] Fig. 6 illustrates an example of the relationship between the system for selecting antibodies and an external server in one embodiment of the present invention.
[Fig. 7] Fig. 7 illustrates an example of a GUI displayed on a monitor in an embodiment of the present invention.
[Fig. 8] Fig. 8 illustrates an example of a GUI displayed in Fig. 7D on a monitor in one embodiment of the present invention.
[Fig. 9] Fig. 9 illustrates an example of a GUI displayed in Fig. 7D on a monitor in one embodiment of the present invention.
[Fig. 10] Fig. 10 illustrates an example of a GUI displayed in Fig. 7D on a monitor in one embodiment of the present invention.
[Fig. 11] Fig. 11 illustrates an example of a GUI displayed on a monitor in one embodiment of the present invention.

### [Embodiments for carrying out the invention]

The objects, features, advantages, and ideas of the present invention are clear to those skilled in the art, and the invention can be easily reproduced from the description herein. The embodiments of the invention and specific examples, etc., described below are shown to illustrate preferred embodiments of the invention and are shown by way of example or explanation, and are not intended to limit the invention thereto. It is clear to those skilled in the art that various changes or modifications can be made based on the description herein within the intent and scope of the invention disclosed herein.

### == Main embodiments of the present inventions ==

[1] A working process of a system for selecting an antibody, the system being communicatively connected to an antibody information server, the system having a control unit and a display device, the working process comprising following steps executed by the control unit: a first step of displaying, on the display device, a microscopic image of biological tissue or cell; a nucleotide sequence of a nucleic acid comprised in the biological tissue or cell; data indicating a characteristics of the nucleic acid or a characteristics of the nucleotide sequence; and name(s) of one or more genes obtained based on the data, a second step of transmitting an ID of a gene selected from the one or more genes to the antibody information server, a third step of receiving information on an antibody against the protein encoded by the selected gene from the antibody information server, a fourth step of displaying the antibody information on the display device.
[2] The working process of Item[1], wherein the one or more genes comprise a first gene obtained based on the data and one or more second genes related to the first gene.
[3] The working process of Item [1] or [2], further comprising a fifth step, executed by the control unit, of ordering the antibody or the production thereof through an Internet to which the system is communicatively connected or the antibody information server.
[4] The working process of Item [3], further comprising a sixth step, executed by the control unit, of displaying an image of a biological tissue or cell stained with the antibody that has been ordered by an orderer in the fifth step and delivered to the orderer.
[5] The working process of Item [3] or [4], further comprising a seventh step, executed by the control unit, of transmitting, to the antibody information server, an image of a biological tissue or cell stained with the antibody that has been ordered by an orderer in the fifth step and delivered to the orderer.
[6] The working process of any one of Items [1] to [5], wherein the antibody information server comprises a storage device for storing a list of the information on one or more antibodies against proteins encoded by a plurality of genes; and the antibody information server receives the ID of the selected gene, identifies the selected gene in the list stored in the storage device, and transmits, to the system, the information on the one or more antibodies linked to the selected gene.
[7] The working process of any one of Items [1] to [6], wherein the first step comprises a sub-step, executed by the control unit, of receiving the microscopic image through the Internet.
[8] The working process of any one of Items [1] to [6], wherein the system further comprises a microscope device; and the first step comprises a sub-step of receiving the microscopic image from the microscopic device.
[9] The working process of any one of Items [1] to [8], wherein the first step comprises a sub-step, executed by the control unit, of receiving the nucleotide sequence of the nucleic acid through the Internet.
[10] The working process of any one of Items [1] to [9], wherein the system further comprises a sequencing device, and the first step comprises a sub-step, executed by the control unit, of receiving the nucleotide sequence of the nucleic acid from the sequencing device.
[11] The working process of any one of Items [1] to []6, [8] and [9], wherein the system further comprises a sequencing device, and the first step comprises a sub-step, executed by the control unit, of receiving the nucleotide sequence of the nucleic acid from the sequencing device and the nucleotide sequence of the nucleic acid is a nucleotide sequence of a nucleic acid extracted from the biological tissue or cell.
[12] The working process of any one of Items [1] to [11], wherein the first step comprises a sub-step, executed by the control unit, of receiving the data indicating the characteristics of the nucleic acid or the characteristics of the nucleotide sequence through the Internet.
[13] The working process of any one of Items [1] to [12], wherein the system further comprises a gene analyzer, and the first step comprises a sub-step, executed by the gene analyzer, of analyzing the nucleotide sequence of the nucleic acid and obtaining the data indicating the characteristics of the nucleic acid or the characteristics of the nucleotide sequence, and a sub-step, executed by the control unit, of receiving the data indicating the characteristics of the nucleic acid or the characteristics of the nucleotide sequence from the gene analyzer.
[14] The working process of any one of Items [1] to [13], wherein the fourth step comprises a sub-step, executed by the control unit, displaying degree of appropriateness of the antibody on the display device.
[15] The working process of Item [14], wherein the degree of appropriateness is a degree of suitability .
[16] The working process of Items [14] or [15], wherein the fourth step comprises a sub-step, executed by the control unit, displaying, on the display device, an antibody, a degree of appropriateness thereof meeting a predetermined condition based on both the information on the antibody received in the third step and the information on the antibodies obtained through the Internet.
[17] The working process of any one of Items [1] to [16], wherein the characteristics of the nucleic acid or the characteristics of the nucleotide sequence is a mutation of the nucleotide sequence, a modification pattern of the nucleic acid, a higher order structure of the nucleic acid, or a binding state of the nucleic acid with a protein.
[18] The working process of any one of Items [1] to [17], wherein the information of the antibody against the protein encoded by the selected gene comprises an image of a biological tissue or cell stained with the antibody.
[19] A program for selecting an antibody, the program causing the system to perform the working process of any one of Items [1] to [18].
[20] A storage medium containing the program of Item [19], the program being stored in a computer readable format.
[21] A system for selecting an antibody, the system communicably connected to the Internet and communicably connected to an antibody information server, the system comprising a control unit and a display device, the control unit displaying on the display device, a microscopic image of a biological tissue or cell, and a nucleotide sequence of a nucleic acid comprised in the biological tissue or cell, and data indicating the characteristics of the nucleic acid or the characteristics of the nucleotide sequence, and name(s) of one or more genes obtained based on the data; transmitting an ID of a gene selected from the one or more genes to the antibody information server; receiving information on an antibody against the protein encoded by the selected gene from the antibody information server; and displaying information on the antibody on the display device.
[22] A system for ordering an antibody or production thereof, the system being communicatively connected to the Internet and communicatively connected to an antibody information server, the system comprising a control unit and a display device, the control unit displaying on the display device, a microscopic image of a biological tissue or cell, and a nucleotide sequence of a nucleic acid comprised in the biological tissue or cell, and data indicating the characteristics of the nucleic acid or the characteristics of the nucleotide sequence, and name(s) of one or more genes obtained based on the data; transmitting an ID of a gene selected from the one or more genes to the antibody information server; receiving information on an antibody against the protein encoded by the selected gene from the antibody information server; displaying information on the antibody on the display device; and ordering the antibody or production thereof through the Internet or the antibody information server.

### == System for selecting antibodies and working process of the system ==

The working process of a system for selecting antibodies disclosed herein (hereinafter often referred to as an antibody selection system or simply as the system), which is communicatively connected to an antibody information server, the system having a control unit and a display device, the working process comprising following steps executed by the control unit: a first step of displaying, on the display device, a microscopic image of biological tissue or cell; a nucleotide sequence of a nucleic acid comprised in the biological tissue or cell; data indicating a characteristics of the nucleic acid or a characteristics of the nucleotide sequence; and name(s) of one or more genes obtained based on the data, a second step of transmitting an ID of a gene selected from the one or more genes to the antibody information server, a third step of receiving information on an antibody against the protein encoded by the selected gene from the antibody information server, a fourth step of displaying the antibody information on the display device. Typically, the control unit is a CPU and the display device is a monitor.

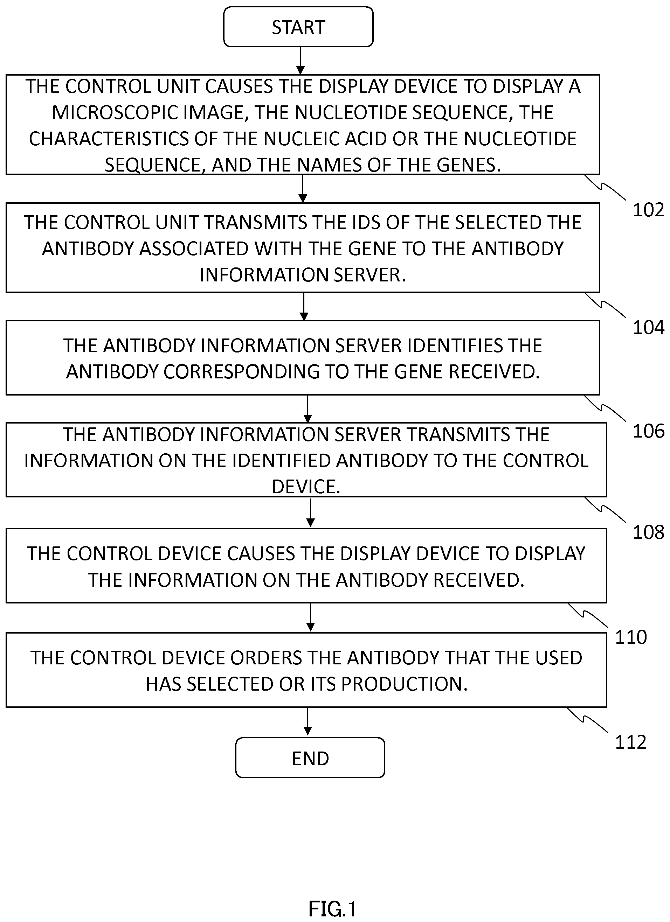

Each step is described in detail below with reference to Figure 1.

### <First Step>

First, the control unit causes the display device to display a microscopic image of the biological tissue or cells, the nucleotide sequence of the nucleic acid possessed by the biological tissue or cells, data indicating the characteristics of the nucleic acid or the characteristics of the nucleotide sequence, and the name(s) of one or more genes obtained based on the data (102).

The microscopic image of the biological tissue or cells is not limited and may be a phase contrast image showing cell morphology, a bright field image of cells stained by, e.g. HE staining, or a fluorescent image of cells stained with an antibody. The state of the biological tissue or cells is also not particularly limited and may be tissue culture or cell culture, or tissue sections such as frozen sections or paraffin sections.

The microscopic images may be received by the control unit through the Internet, or the system may have a microscope device and receive the images from the microscope device. In the latter case, the user prepares biological tissue or cells, observes them under a microscope, and transmits the images captured using a CCD camera or similar device to the control unit.

The nucleic acid possessed by the biological tissue or cells is a nucleic acid possessed by at least a part of cells of the biological tissue or cells in the microscopic image. It is not limited as long as t it is be derived from the same type of cells as those in the image; it may be a nucleic acid extracted from the cells themselves appearing in the image or from cells of the same type as but different from those in the image. The nucleic acid may be DNA or RNA.

The nucleotide sequence of the nucleic acid may be the entire nucleotide sequence of the nucleic acid or a desired portion of the nucleotide sequence. This nucleotide sequence may be obtained by the control unit through the Internet, or the system may further have a nucleotide sequence analyzer to analyze the nucleotide sequence of the desired nucleic acid and transmit it to the control unit. The nucleic acid to be analyzed by the sequencer may be extracted from at least some of the cells of the biological tissue or cells used to produce the microscopic image, or from cells of the same species as but different from the cells in the image. The control unit of the system may also use nucleotide sequences that another device or system has obtained and stored from the Internet or a nucleotide sequence analyzer.

As used herein, "the same type" means "the same cell-type". The nucleic acid may be obtained from the same tissue of the same individual as the biological tissue or cells used to produce the microscopic image; from the same tissue of a different individual of the same species; from the same tissue of an individual of a different species; or from a homologous tissue of an individual of a different species.

Data indicating characteristics of the nucleic acid or characteristics of the nucleotide sequence may include, raw data, analytical data, or data from investigation indicating the physical and chemical characteristics of the nucleic acid or its sequence, such as mutations in the nucleotide sequence (deletions, substitutions, insertions, or SNPs), modification patterns of the nucleic acid (e.g., frequency and pattern of cytosine methylation), higher order structure of the nucleic acid (e.g., hairpin structure), protein binding status (e.g., histone binding levels).

Data indicating characteristics of nucleic acids or nucleotide sequences may be data obtained through the Internet by searching based on nucleotide sequences of nucleic acids, or the system may further have a gene analyzer to analyze the structure of nucleic acids, nucleotide sequences of nucleic acids, interactions between nucleic acids and proteins, etc. to obtain data indicating characteristics of nucleic acids or characteristics of nucleotide sequences. The control unit of the system may also use data which another device or system obtained from the Internet or a nucleotide sequence analyzer and stored therein.

One or more genes obtained based on data indicating characteristics of the nucleic acid or the nucleotide sequence means genes that are directly or indirectly related to the characteristics of the nucleic acid or the nucleotide sequence. For example, a directly related gene means a gene that partially or wholly contains a sequence having the characteristics. An indirectly related gene is a gene that is related to a directly related gene, for example, a gene whose expression is controlled by the directly related gene, a gene that is genetically upstream or downstream of the directly related gene, a gene encoding a protein upstream or downstream of a protein encoded by the directly related gene in a signal transduction pathway, a gene encoding a protein that binds to a sequence having that characteristic, etc. Here, "gene" shall mean a coding region and its expression control region. The expression control region may be upstream or downstream of the coding region. The name of a gene means a notation that can indicate and identify an individual gene or a part thereof, or a set of several genes, and may be represented by an ID such as a code or a number. No gene may be obtained and be indicated based on data indicating characteristics of the nucleic acid or the nucleotide sequence.

The nucleotide sequence analyzer or gene analyzer may be connected to the control unit via the Internet or directly via LAN or other means. The nucleotide sequence analyzer and genetic analyzer may be installed in an analysis facility that is independent of this system.

### <Second step>

Next, from the one or more genes obtained based on data indicating characteristics of the nucleic acid or the nucleotide sequence in the first step, the user selects one or more genes encoding proteins against which the user desires to obtain an antibody. The control unit transmits the IDs of the selected genes to the antibody information server, which receives the IDs (104).

The antibody information server and the system may be connected via the Internet or directly via LAN.

The antibody information server preferably has a storage device, and the storage device may store a list of one or more antibodies against proteins encoded by various genes and information about them. This information may include the epitopes recognized by the antibodies, images of samples such as biological tissue or cells stained with the antibodies, the companies selling the antibodies, the characteristics of the antibodies (monoclonal or polyclonal, what species of animal they were created from, what fluorescent dye they are conjugated to, suitable uses, etc.), and names of the documents (e.g., patent documents, scientific papers, etc.) that describe experiments using the antibody.

]

### <Third step>

Next, the antibody information server identifies the gene corresponding to the gene ID received in the second step, selects the information on the antibody associated with the gene from the list (106), and transmits it to the system as the antibody information (108). If there is no antibody associated with the gene corresponding to the gene ID received in the second step, the system sends, to the system, information that the antibody for that gene does not exist in the list. The control unit of the system thus receives from the antibody information server information about antibodies against the protein encoded by the selected gene.

### <Fourth step>

The control unit causes the display device to show the gene name associated with the ID sent to the antibody information server and the information on the antibody received from the antibody information server (110). When there are no antibody associated to the gene corresponding to the gene ID received in the second process, the display may show "No antibody" or the like. When there is no specific information on the antibody, it may be displayed as "There is no specific information" or the like.

### <Fifth step>

The information on the antibody that the control unit obtains from the antibody information server includes the presence or absence of the antibody and, when the antibody exists, the company selling the antibody and the characteristics of the antibody. When there are multiple antibodies, the user can select the antibody suitable for his/her own use based on the antibody's characteristics and other information. The selected antibody can then be ordered using the control unit to the company selling the antibody through the Internet or antibody information server to which the system is communicatively connected (112). When the antibody does not exist, the system can order the production of an antibody for the desired gene from a company that produces antibodies.

The evaluation of antibodies to select the antibodies or the selection of the antibodies based on the evaluation may be automated.

For example, the control unit may score appropriateness of the antibodies based on the matching conditions of use, past purchase history that was fed back, evaluations from buyers that were fed back, past purchase frequency, and literature information, which have been obtained as information on the antibodies from the antibody information server, and display them with the score on the display device, arranged from the product with the highest score to the product with the lowest score. A threshold of appropriateness may be set for the score, and recommendations may be displayed for one or more antibodies that exceed the threshold. The appropriateness refers to the degree to which a person skilled in the art can judge that the antibody is appropriate in all aspects as an antibody to be used. For example, the degree of appropriateness may be based on the degree of conformity, which indicates the degree of conformity with the user's requirements for the antibody. In such a case, the user may input factors such as the purpose and use of the antibody, and then the degree to which each antibody is compatible with those factors may be evaluated. The user may input the importance of each piece of information to the user's use, and the control unit may consider the importance as a weight and evaluate the antibodies. In these cases, since a particularly useful evaluation for selecting suitable antibodies is the frequency of past purchases, the user may evaluate the antibodies by other factors, and after selecting several antibodies with scores higher than the threshold, the user may rate them as suitable in order of frequency of past purchases. Such a method allows the user to select the antibodies that are considered most appropriate for his/her purposes. The control unit may automatically select the antibodies with the highest appropriateness scores and present them to the user. In this case, the user may not consider only the score, but the user may select the antibody with the highest frequency of past purchases among antibodies with a score above a threshold value.

The literature information is for literature in which the antibody is used, preferably the literature in which the stained image is published. When the literature is an article, it may be the impact factor of the journal in which the article is published or the number of times the article has been cited. Here, the literature in which the antibody is used and the literature in which its stained image is published may be analyzed using AI.

The quality of the antibody may be scored using AI based on the photographs in which the antibody is used. For example, a stained image with high background and a stained image with low background can be trained as training data, and a score can be obtained to evaluate how much background the stained image obtained as described above has.

The evaluation of antibodies for selecting antibodies may be based not only on information about antibodies obtained by the control unit from an antibody information server, but also on information about antibodies obtained through the Internet. For example, stained images can be obtained from company websites or personal SNS sites for literature information.

### <Sixth step>

Meanwhile, the user can stain the biological tissue or cells using the antibodies obtained by ordering. The control unit can then cause the display device to show an image of the biological tissue or cells thus stained.

Using this system in this way, based on the characteristics of the nucleic acid and its nucleotide sequence, antibodies against proteins encoded by genes related to the nucleic acid and its nucleotide sequence can be easily purchased and the expression of the genes can be easily examined.

### <Seventh step>

The control unit may transmit an image of the biological tissue or cell stained with the antibody ordered in the fifth step and delivered to the orderer in the sixth step as feedback to the antibody information server. This will allow other users connected to the antibody information server to share the results as reference data.

### <Eighth step>

At any point in the second to seventh steps, the user may re-select one or more genes obtained based on the data indicating characteristics of the nucleic acid or the characteristics of the nucleotide sequence in the first step, which are different from the genes selected in the second step. In such a case, for the newly re-selected gene, the process can be repeated from the stage where the control unit transmits the ID of the newly selected gene to the antibody information server in the second step to the seventh step.

### <Ninth step>

At some point between the fifth step and the seventh step, from the antibodies displayed in the fourth process, the antibodies different from those selected in the fifth step can be re-selected. In such a case, the process should be repeated from the ordering in the fifth step to the seventh step for the newly re-selected antibody.

### == Program and storage medium ==

An embodiment disclosed herein is a program for selecting antibodies, which causes the system to perform any of the methods described above. A storage medium containing the program in a computer readable format is another embodiment of the invention.

### == relationship between a system for selecting antibodies and an external server ==

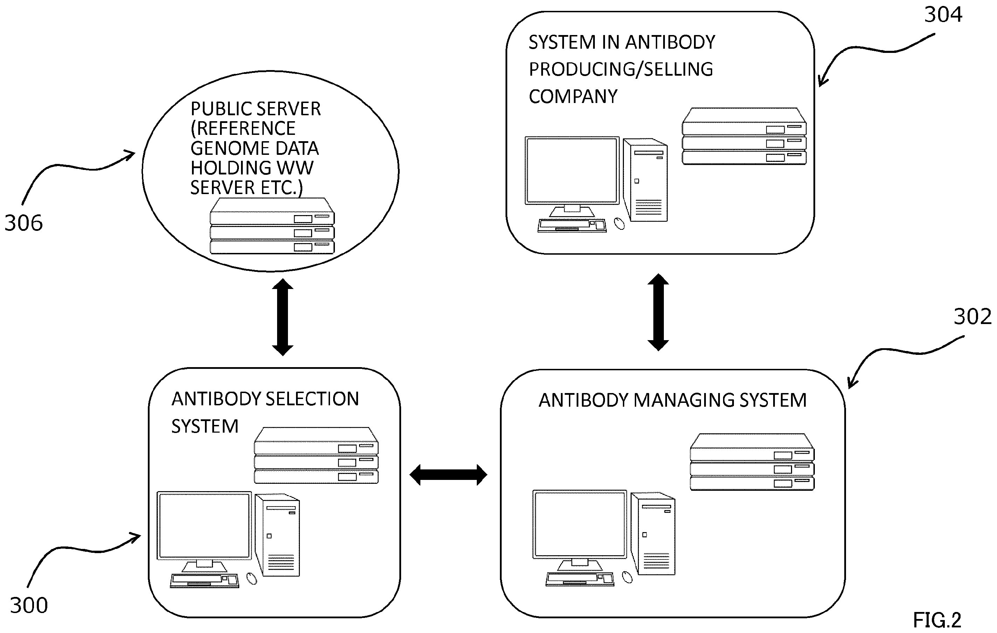

The relationship between the system disclosed herein and an external server is described in detail below with reference to Figures 2-6. Here, an external server is a server to which the antibody managing system and/or the antibody selection system is communicatively connected via the Internet and which has functions related to the system. External servers include publicly available servers that can be used for free or for a fee, and examples include genebanks such as EMBL, protein banks, and article search engines such as PubMed.

### (1) A system for selecting antibodies, and the overall picture including other systems related to the system and external servers

First, the overall picture, including the system for selecting antibodies and other systems related to the system and external servers, is described with reference to Figure 2.

A user who wishes to order antibodies has the antibody selection system 300 disclosed herein. This antibody selection system 300 is connected to the antibody managing system 302 via the Internet. This antibody managing system 302 is connected, via LAN or the Internet, to a system 304 that manages antibody information in antibody manufacturing companies and/or antibody selling companies, and manages information (e.g., antibody details and number of antibodies in stock at each company) on antibodies manufactured and/or sold by the companies. The antibody selection system 300 is also connected, via the Internet, to a reference genome data holding server 306, which allows users to obtain various genome information.

### (2) Basic Configuration of Antibody Selection System

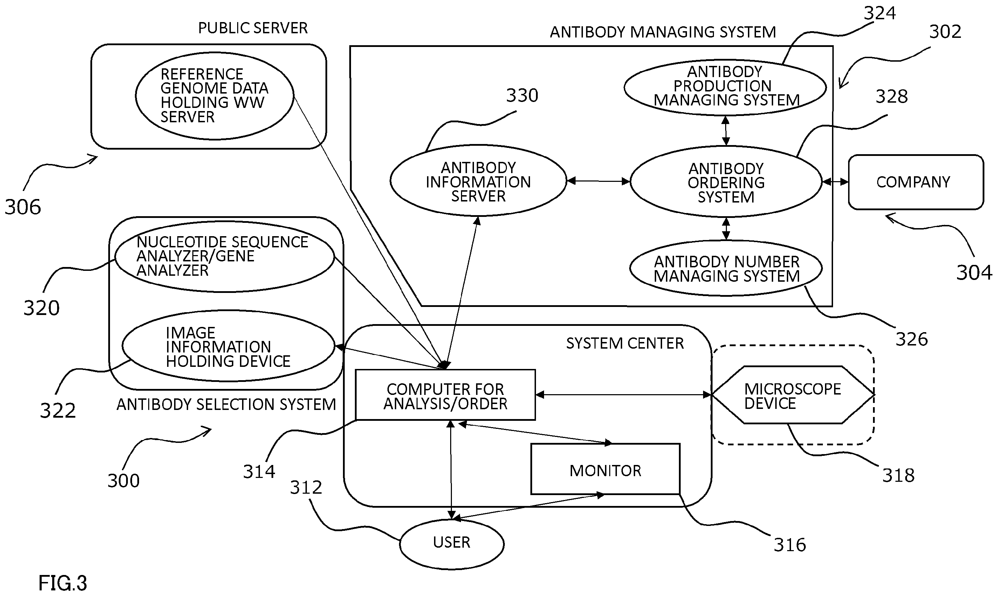

Figures 3 to 6 show examples of specific configurations of the antibody selection system.

First, the basic configuration common to them is described.

The antibody selection system 300 disclosed herein has a computer for analysis/order 314 (hereinafter referred to as "computer") as a control unit and a monitor 316 as a display device. The system may have a microscope device 318. In addition, the system may have a nucleotide sequence analyzer 320, a gene analyzer 320, and an image information holding device 322, which may be an integral device or independently separate devices. The image information holding device 322 is a device that stores images obtained by the microscope device 318 and images obtained via the Internet, and may be a memory such as an external hard disk, an independent computer, or a built-in memory provided in the computer for analysis/order 314. In the figure, the nucleotide sequence analyzer 320 and the gene analyzer 320 are configured as an integrated device, and the image information storage device 322 is provided as an independent device. They may be directly connected by LAN to the system center with the computer for analysis/order 314, or they may be independent as analysis facilities and connected to the system center via the Internet, but for example, in the instant configuration, they are directly connected by LAN.

Independent of the antibody selection system 300, an antibody managing system 302 is provided. The antibody managing system 302 may include an antibody production managing system 324, an antibody number managing system 326, and an antibody ordering system 328, which may be solely or jointly owned by antibody sales companies or by antibody management companies. The antibody production managing system 324 manages the production of antibodies sold by a company. For example, when the number of a particular antibody decreases in the antibody number managing system 326, the name of the antibody and the number of antibodies remaining is communicated to the antibody production managing system 324, and the antibody production managing system 324 requests a company 304 or an antibody production system owned by company 304 to begin production of the antibody. When the company 304 produces the antibody and enters that information to the antibody number managing system 326, the antibody number managing system 326 updates the number of antibodies produced. The antibody ordering system 328 receives orders for antibodies or antibody production from the user 312, and the company 304 sells the antibodies to the user 312 according to the order or produces the antibodies according to a request from the user 312. In the figure, the antibody ordering system 328 exclusively communicates with the user, but the antibody production managing system 324 and the antibody number managing system 326 can also be configured to communicate with the user 312. In addition to these systems, the antibody selection system 300 may be communicatively connected through the Internet to a public server 306 such as a gene bank, antibody bank, reference genome data holding WW server, literature search engine, etc.

The antibody selection system or antibody managing system may be provided with a literature analysis section. The literature analysis section may be located anywhere in the antibody selection system or antibody managing system, but preferably in the antibody information server or computer for analysis/order. The literature analysis section is communicatively connected to a literature database, such as a literature search engine outside the antibody selection system or a server of a publisher of literature, and searches literature published on the Internet to collect literature in which a given antibody is used, preferably literature in which images of staining with the antibody are published. Then, based on the impact factor of the journals in which those articles are published, the number of times those articles are cited, etc., the antibody is scored and provided to the user along with the antibody information.

The following are several examples of system configurations that vary depending on where the antibody information server 330 is located.

### (1) The antibody information server 330 resides in the antibody managing system 302.

In Figure 3, the antibody selection system 300 and the antibody information server 330 are connected via the Internet. And the antibody information server 330 and the antibody ordering system 328 are connected via LAN.

The antibody managing system 302 is connected to the system of each antibody selling company 304 to obtain information, thereby centralizing information management.

In this case, the antibody information server 330 can be connected to multiple antibody selection systems 300, thereby connecting to multiple users. Thus, it is easier to accumulate information in the antibody information server 330, e.g., through feedback. The antibody managing system may be in the so-called cloud.

### (2) The antibody information server 330 is connected to the antibody selection system 300 by LAN.

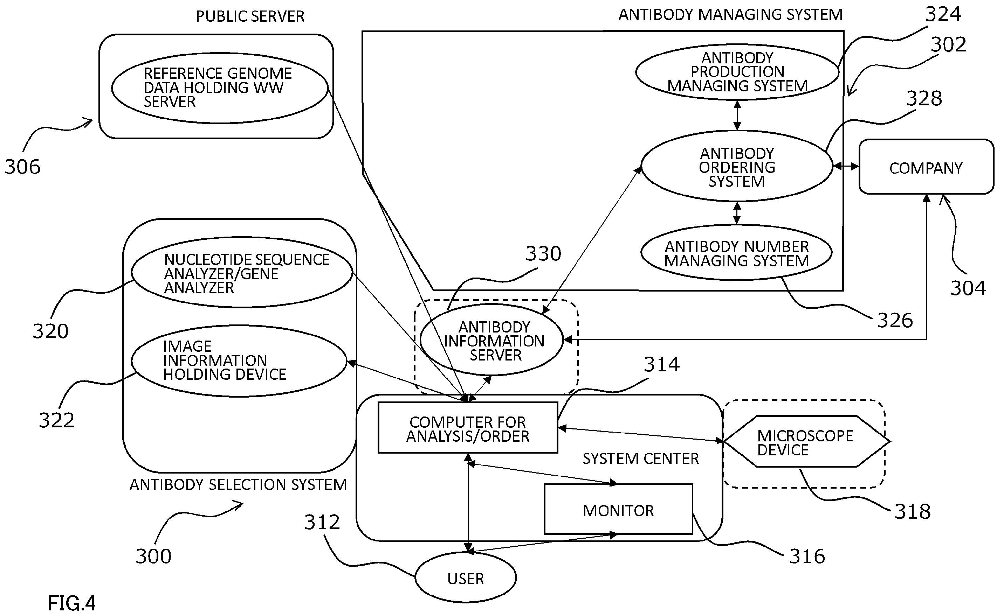

In Figure 4, the antibody selection system 300 and the antibody information server 330 are connected by LAN. And the antibody information server 330 and the antibody ordering system 328 are connected via the Internet.

In this case, the antibody information server 330 is connected to the system of each antibody selling company 304 via the Internet to accumulate information.

The antibody information server 330 of each user 312 is connected to the antibody ordering system 328 and each user 312 can order the required antibody or antibody production.

### (3) The antibody information server 330 is in the antibody ordering system 328.

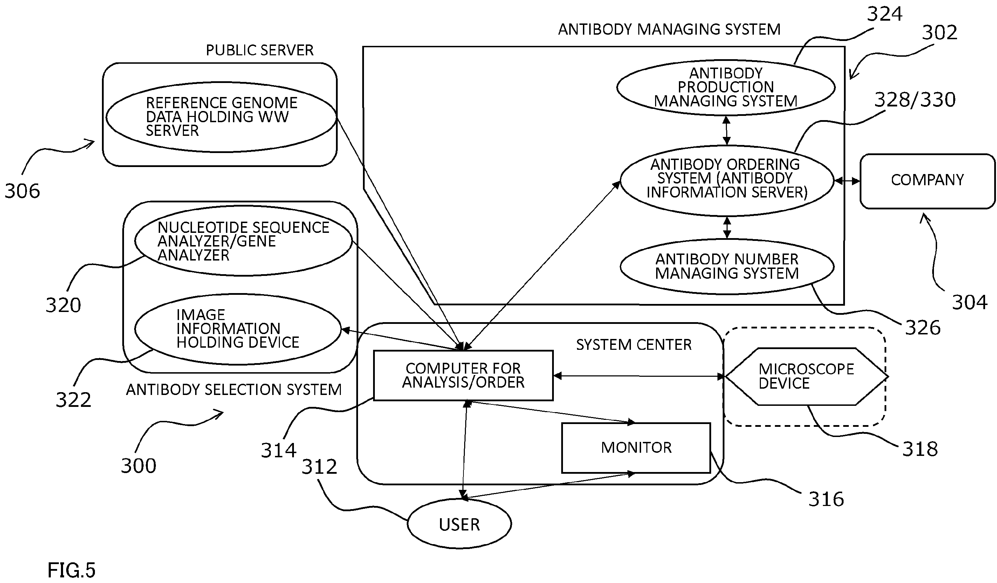

In Figure 5, the antibody information server 330 is in the antibody ordering system 328. Basically, the effect is the same as (1), but by placing the antibody information server 330 in the antibody ordering system 328, the antibody information in the antibody information server 330 can be updated more quickly.

### (4) There are multiple antibody information servers 330, connected to the antibody selection system 300 by LAN and connected to the antibody managing system 302 via the Internet.

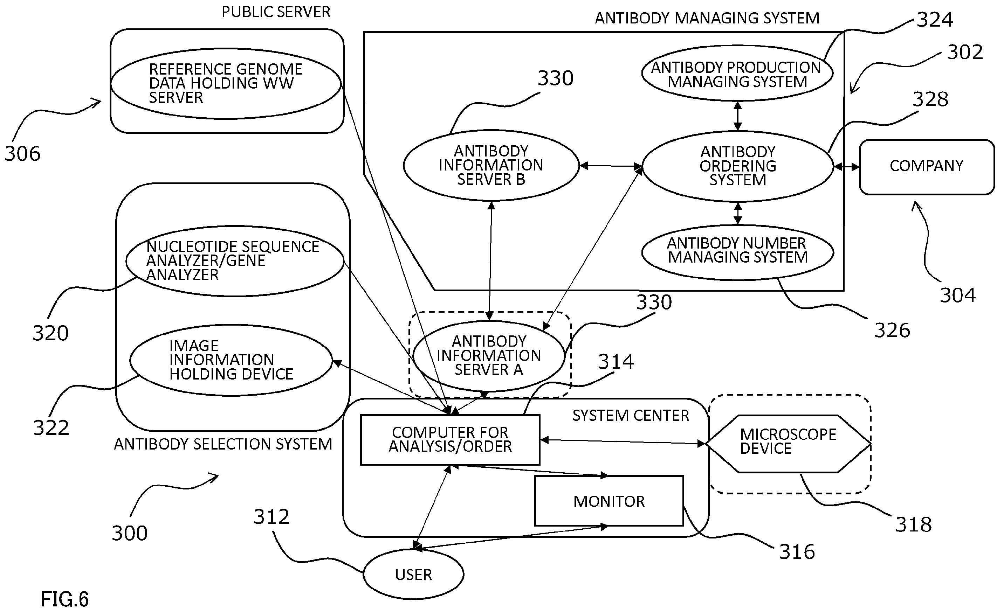

In (2), since the antibody managing system 302 does not have an antibody information server 330, even if the antibody managing system 302 is connected to multiple users it was not easy to receive feedback from the users. In the configuration shown in Figure 6, since the antibody information server A330 is connected to the antibody selection system by LAN and the antibody information server B330 exists in the antibody managing system 302, the users can freely manage antibody information using the antibody information server A. In addition, by being connected to multiple users 312, the antibody information server B can accumulate feedback from the users 312.

In this case, each antibody information server A 330 and antibody information server B 330 should have a storage device that stores a list of information on one or more antibodies against proteins encoded by a plurality of genes. Antibody information server B330 creates the list with information from companies 304. On the other hand, antibody information server A330 may receive information from companies and create the list, but it is preferred to receive information from antibody information server B330 and create the list.

### = GUI =

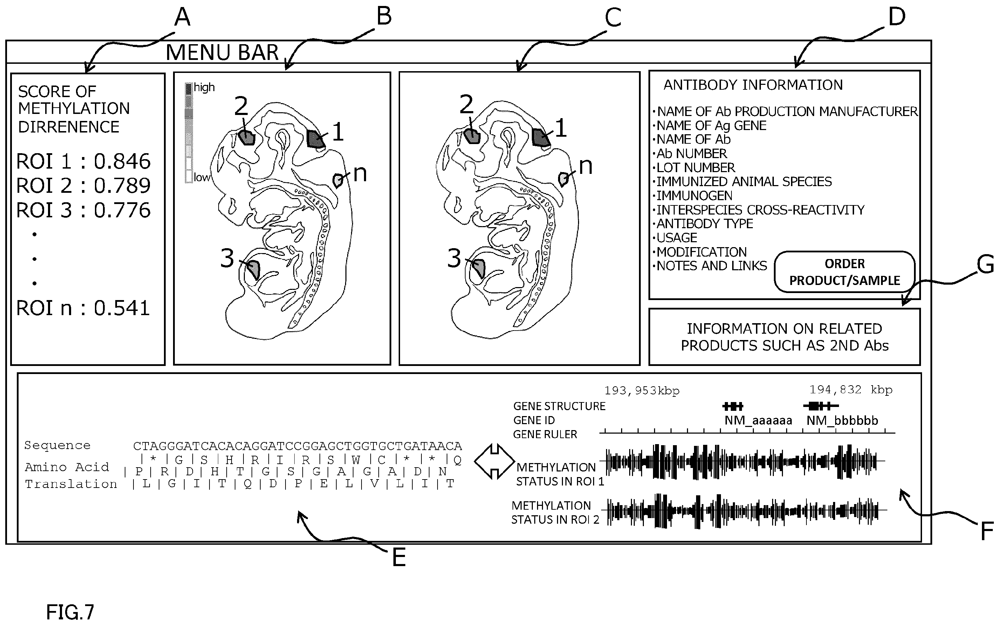

An example of the GUI on the monitor of the antibody selection system is shown in Figure 7. Figure 7 shows an example of analyzing methylation in a DNA sequence extracted from a specific region in a microscopic image of a biological tissue or cells. The system is controlled by a computer.

Figure 7b shows a microscopic image of a biological tissue or cells. In this example, a brightfield image of a HE-stained tissue section of a mouse embryo, acquired by the user using microscope device 318, is displayed.

The user collects cells in each region (ROI 1-n) from this tissue section, isolates genomic DNA from the collected cells, and determines the nucleotide sequence using a sequencer (nucleotide sequence analyzer/gene analyzer 320). The data of the nucleotide sequence in the designated chromosomal region and the amino acid sequence deduced from the nucleotide sequence is displayed on a monitor (Figure 7E).

At the same time, for DNAs obtained from cells in different ROIs, the methylation of cytosine in the regions displayed on the monitor as a gene ruler (upper part of Figure 7F) is analyzed, the results are analyzed by computer, scores for methylation are calculated and the scores are displayed on the monitor as graphs (lower part of Figure 7F).

In the method of calculating the score related to methylation, for example, the methylation frequency, i.e., the percentage of bases of a particular type detected as methylated in a particular position or region on the genome, or the methylation amount, i.e., the amount of methylation detected in a particular position or region on the genome may be calculated as the score. Alternatively, change in the percentage of methylation or in the amount of methylation may be monitored as the score.

In a particular genomic DNA region, a score for methylation of nucleic acids is determined for each ROI and the ROIs are arranged in order of increasing value (Figure 7A). At the same time, the methylation score may also be reflected and visualized on the image. For example, according to the methylation score, the corresponding ROIs are colored and displayed in a heat map format overlaid on a brightfield image of a HE-stained tissue section of a mouse embryo (Figure 7B). Here, for example, the user may select ROI 1 with the largest score.

In the upper part of Figure 7F, along the gene ruler, the names of the genes and their IDs that are present in the described DNA region, genes on or near the nucleotide sequence, genes that are genetically downstream of the genes in the DNA region, genes encoding proteins that bind to the nucleotide sequence, etc., are listed at the relevant sequence location.

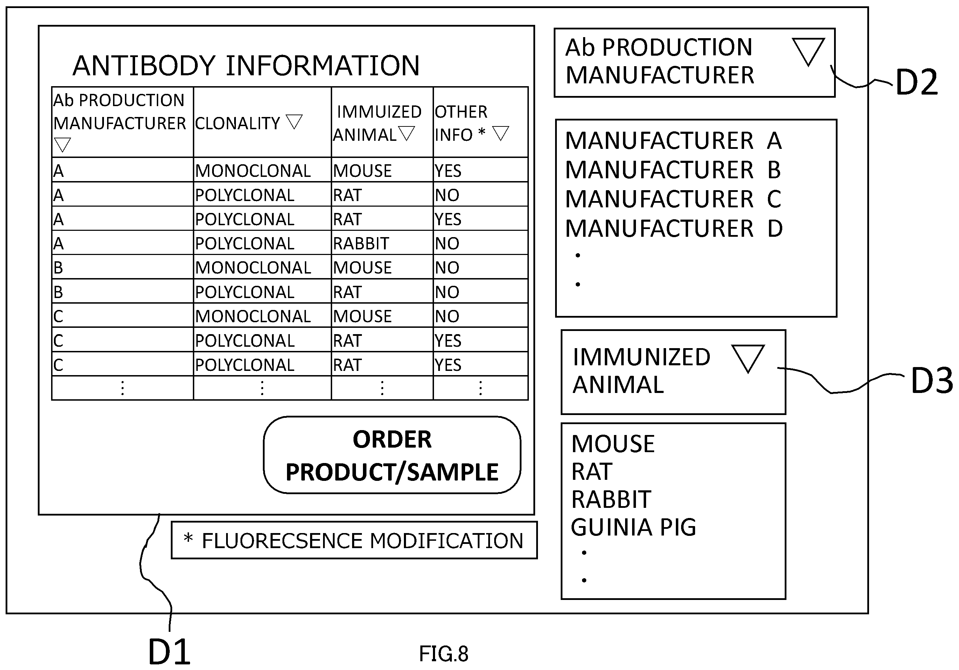

When the user selects a gene of interest among those genes and clicks on the gene name, a list of commercially available antibodies linked to the gene name, sent from the antibody information server, is displayed at the position shown in Figure 7D, as shown in Figure 8. Thereafter, when the user selects the gene again, the list of antibodies against the newly selected gene will be displayed in the same manner.

In Figure 8, for each antibody, information such as antibody manufacturer, antibody clonality, immunized animal species, and fluorescent and enzymatic modifications are available. All or part of these lists may be displayed in a pull-down format.

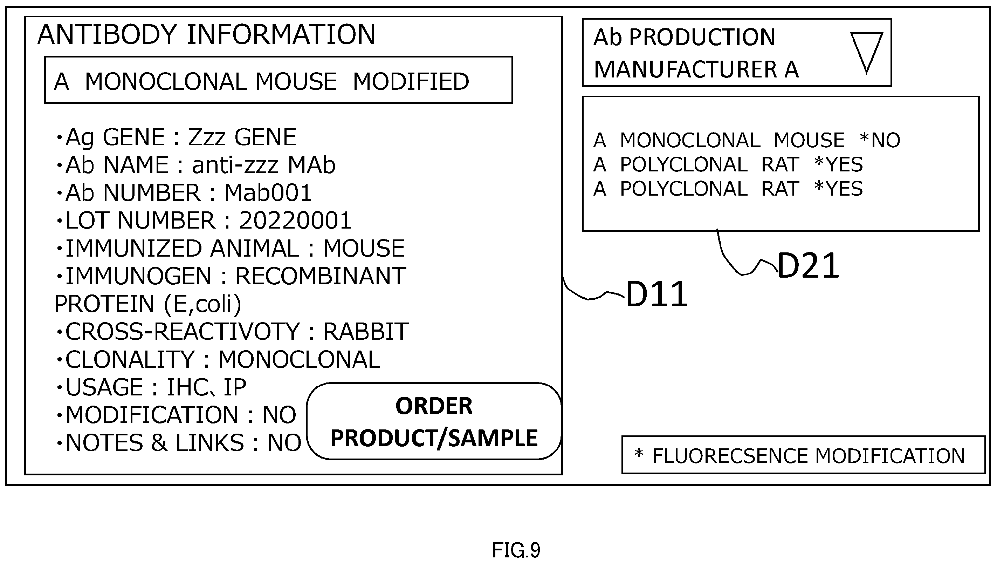

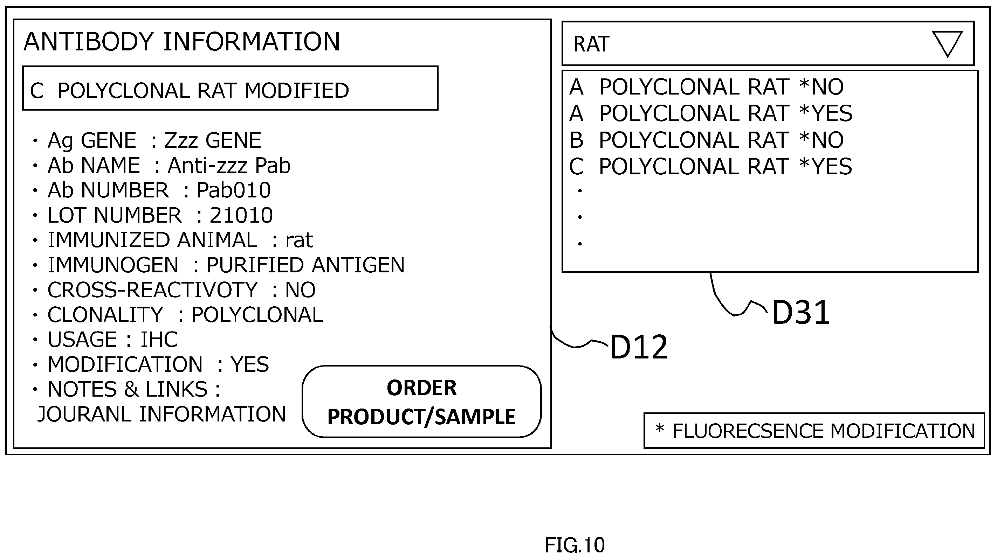

When the user clicks on the column of the antibody of interest in List D1 in Figure 8, details of the antibody are displayed, as shown in List D11 in Figure 9 and List D12 in Figure 10.

When the user clicks on the antibody manufacturer of interest (e.g., Company A) in List D2 in Figure 8, a list of antibodies produced by the same antibody manufacturer (in this case, Company A) is displayed, as shown in List D21 in Figure 9. When the user clicks on the antibody of interest (e.g., a mouse monoclonal antibody) in list D21 in Figure 9, the list in Figure 9D11 will display the details of the clicked antibody.

Next, if the user clicks on the immunized animal species of interest (e.g., rat) in list D3 of Figure 8, a list of antibodies that have been produced in the same immunized animal species (here, rat) will be displayed, as shown in list D31 of Figure 10. When the user clicks on the antibody of interest (e.g., manufacturer C's antibody) in list D31 in Figure 10, the list in Figure 10D12 will display details of the clicked antibody.

In this embodiment, the antibody manufacturer or immunized animal species are used as examples, but the antibodies may be classified by experimental use (e.g., tissue staining, immunoprecipitation, Western blotting, etc.), antibody species (e.g., IgM, IgG, etc.), modifier (e.g., fluorescent dye, enzyme, etc.), etc., and these may be used as indicators to make a sublist of antibodies such as List D21 and List D31. A sublist with two indicators may be made by extracting those with the second indicator from the sublist made with the first indicator.

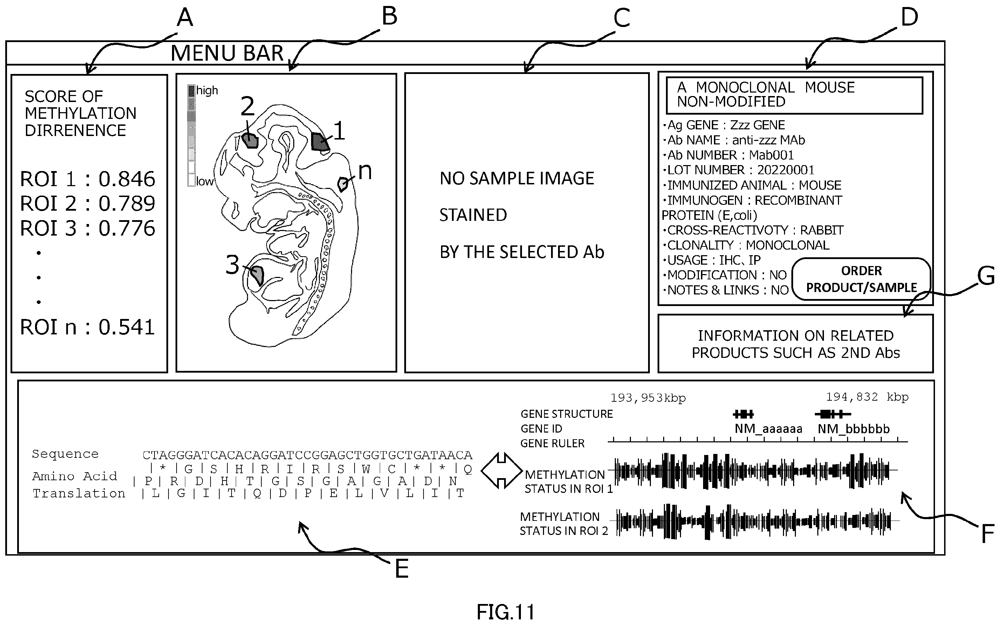

Thus, when an interested antibody is selected from the antibody list and clicked, the antibody information server sends detailed information about that antibody, as shown in Figure 7D. At the same time, if a stained image with that antibody is included in the antibody information, it will be displayed in Figure 7C. If the stained image by that antibody is not included in the information, it may be displayed as shown in Figure 11, e.g., "There is no stained sample image for the selected antibody."

If the user re-selects an antibody from the antibody list, detailed information about the newly selected antibody will be displayed in the same manner.

Next, when the user finds an antibody from the list of commercially available antibodies that he/she would like to purchase or for which he/she would like its sample, he/she can click on the icon in Figure 7D labeled "Order/Sample Request" to select whether to order the antibody or to request a sample of the antibody, and thus the antibody or sample can be ordered. At the same time, Figure 7G shows a list of reagents (such as secondary antibodies or enzyme substrates) that would be useful in using the antibody, and the user can use that information for the actual test.

### [Industrial Availability]

The present invention can provide a system for easily selecting antibodies and a working process thereof.

### [description of code]

- 300:: Antibody selection system
- 302:: Antibody managing system
- 304:: Company
- 306:: Public server
- 312:: User
- 314:: Computer for analysis/order
- 316:: Monitor
- 318:: Microscope device
- 320:: Gene analyzer
- 322:: Image information holding device
- 324:: Antibody production managing system
- 326:: Antibody number managing system
- 328:: Antibody ordering system
- 330:: Antibody information server

## Claims

1. A working process of a system for selecting an antibody, the system being communicatively connected to an antibody information server, the system having a control unit and a display device, the working process comprising following steps executed by the control unit:
a first step of displaying, on the display device,
a microscopic image of biological tissue or cell;
a nucleotide sequence of a nucleic acid comprised in the biological tissue or cell;
data indicating a characteristics of the nucleic acid or a characteristics of the nucleotide sequence; and
name(s) of one or more genes obtained based on the data,
a second step of transmitting an ID of a gene selected from the one or more genes to the antibody information server,
a third step of receiving information on an antibody against the protein encoded by the selected gene from the antibody information server,
a fourth step of displaying the antibody information on the display device.

2. The working process of Claim 1, wherein the one or more genes comprise a first gene obtained based on the data and one or more second genes related to the first gene.

3. The working process of Claim 1 or 2, further comprising a fifth step, executed by the control unit, of ordering the antibody or the production thereof through an Internet to which the system is communicatively connected or the antibody information server.

4. The working process of Claim 3, further comprising a sixth step, executed by the control unit, of displaying an image of a biological tissue or cell stained with the antibody that has been ordered by an orderer in the fifth step and delivered to the orderer.

5. The working process of Claim 3 or 4, further comprising a seventh step, executed by the control unit, of transmitting, to the antibody information server, an image of a biological tissue or cell stained with the antibody that has been ordered by an orderer in the fifth step and delivered to the orderer.

6. The working process of any one of Claims 1-5, wherein
the antibody information server comprises a storage device for storing a list of the information on one or more antibodies against proteins encoded by a plurality of genes; and
the antibody information server receives the ID of the selected gene, identifies the selected gene in the list stored in the storage device, and transmits, to the system, the information on the one or more antibodies linked to the selected gene.

7. The working process of any one of Claims 1-6, wherein
the first step comprises a sub-step, executed by the control unit, of receiving the microscopic image through the Internet.

8. The working process of any one of Claims 1 to 6, wherein
the system further comprises a microscope device; and
the first step comprises a sub-step of receiving the microscopic image from the microscopic device.

9. The working process of any one of Claims 1 to 8, wherein
the first step comprises a sub-step, executed by the control unit, of receiving the nucleotide sequence of the nucleic acid through the internet.

10. The working process of any one of Claims 1 to 9, wherein
the system further comprises a sequencing device, and the first step comprises a sub-step, executed by the control unit, of receiving the nucleotide sequence of the nucleic acid from the sequencing device.

11. The working process of any one of Claims 1 to 6, 8 and 9, wherein
the system further comprises a sequencing device, and the first step comprises a sub-step, executed by the control unit, of receiving the nucleotide sequence of the nucleic acid from the sequencing device and
the nucleotide sequence of the nucleic acid is a nucleotide sequence of a nucleic acid extracted from the biological tissue or cell.

12. The working process of any one of Claims 1 to 11, wherein
the first step comprises a sub-step, executed by the control unit, of receiving the data indicating the characteristics of the nucleic acid or the characteristics of the nucleotide sequence through the Internet.

13. The working process of any one of Claims 1 to 12, wherein
the system further comprises a gene analyzer, and
the first step comprises
a sub-step, executed by the gene analyzer, of analyzing the nucleotide sequence of the nucleic acid and obtaining the data indicating the characteristics of the nucleic acid or the characteristics of the nucleotide sequence, and
a sub-step, executed by the control unit, of receiving the data indicating the characteristics of the nucleic acid or the characteristics of the nucleotide sequence from the gene analyzer.

14. The working process of any one of Claims 1 to 13, wherein
the fourth step comprises a sub-step, executed by the control unit, displaying degree of appropriateness of the antibody on the display device.

15. The working process of Claim 14, wherein the degree of appropriateness is a degree of suitability .

16. The working process of claims 14 or 15, wherein
the fourth step comprises a sub-step, executed by the control unit, displaying, on the display device, an antibody, a degree of appropriateness thereof meeting a predetermined condition based on both the information on the antibody received in the third step and the information on the antibodies obtained through the Internet.

17. The working process of any one of Claims 1-16, wherein
the characteristics of the nucleic acid or the characteristics of the nucleotide sequence is a mutation of the nucleotide sequence, a modification pattern of the nucleic acid, a higher order structure of the nucleic acid, or a binding state of the nucleic acid with a protein.

18. The working process of any one of Claims 1-17, wherein
the information of the antibody against the protein encoded by the selected gene comprises an image of a biological tissue or cell stained with the antibody.

19. A program for selecting an antibody, the program causing the system to perform the working process of any one of Claims 1-18.

20. A storage medium containing the program of Claim 19, the program being stored in a computer readable format.

21. A system for selecting an antibody, the system communicably connected to the Internet and communicably connected to an antibody information server,
the system comprising a control unit and a display device, the control unit
displaying on the display device,
a microscopic image of a biological tissue or cell, and
a nucleotide sequence of a nucleic acid comprised in the biological tissue or cell, and
data indicating the characteristics of the nucleic acid or the characteristics of the nucleotide sequence, and
name(s) of one or more genes obtained based on the data;
transmitting an ID of a gene selected from the one or more genes to the antibody information server;
receiving information on an antibody against the protein encoded by the selected gene from the antibody information server; and
displaying information on the antibody on the display device.

22. A system for ordering an antibody or production thereof, the system being communicatively connected to the Internet and communicatively connected to an antibody information server,
the system comprising a control unit and a display device,
the control unit displaying on the display device,
a microscopic image of a biological tissue or cell, and
a nucleotide sequence of a nucleic acid comprised in the biological tissue or cell, and
data indicating the characteristics of the nucleic acid or the characteristics of the nucleotide sequence, and
name(s) of one or more genes obtained based on the data;
transmitting an ID of a gene selected from the one or more genes to the antibody information server;
receiving information on an antibody against the protein encoded by the selected gene from the antibody information server;
displaying information on the antibody on the display device; and
ordering the antibody or production thereof through the internet or the antibody information server.
